# EUROPEAN PATENT APPLICATION

(11) **EP 1 258 532 A1**
(43) Date of publication of application: **20.11.2002**
(21) Application number: 01110040.1
(22) Date of filing: 26.04.2001
(51) Int. Cl.: C12N 15/56, C12N 9/86, C12N 15/63, C12N 15/70, C12P 13/04, C12P 41/00

(54) **Thermostable D-hydantoinase from Bacillus circulans and the application thereof**

(71) Applicant: NATIONAL SCIENCE COUNCIL, Taipei (TW)
(72) Inventor: Hsu, Wen-Hwei, Taichung (TW); Kao, Chao-Hung, Taipei (TW)
(74) Representative: Benedum, Ulrich Max, Dr.

(57) **Abstract**

The invention discloses a thermostable D-hydantoinase from Bacillus circulans, and relates to the nucleic acid sequence, amino acid sequence and vector constructs of the enzyme. The thermostable D-hydantoinase of the invention shows about 45%-70% identity in amino acid sequence with other D-hydantoinases. The thermostable D-hydantoinase of the invention converts 5'-substituted D-hydantoinase to the corresponding N-carbamoyl-D- and/or -L-α/β-amino acids, and retains at least 50% activity after 30 days at 50°C. In addition, the enzyme activity can also enhanced by certain divalent cations.

## Description

### BACKGROUND OF THE INVENTION

### Field of the Invention

The present invention relates to a novel thermostable D-hydantoinase, and particularly to the nucleic acid sequence, amino acid sequence, vector constructs and the application thereof.

### Description of the Related Arts

D-amino acids are usually called unnatural amino acids. They are not used as frequently as L-amino acids, but are becoming more and more important, especially in the field of pharmacy, e.g. in the preparation of penicillin, cephalosporin and β-lactam antibiotic precursors.

Amoxicillin can be obtained by reacting D-*p*-Hydroxy-phenylglycine (D-*p*-HPG) with 6-amino-penicillianic acid. Recently, the market for amoxicillin has expanded and grown more than 12% with each year. Due to the enhanced sales volume of the products, the demands for D-*p*-HPG precursors have increased markedly, with growth over 10% each year.

D-*p*-HPG was once chemically synthesized from D,L-*p*-hydroxyphenylhydantoin (D,L-*p*-HPH). However, this method has numerous disadvantages, including: (1) many reaction steps are required; (2) expensive solvents are employed; (3) products with low optical purity are obtained; (4) low yield is obtained in each reaction cycle; (5) complex steps are required for separation of racemizing residues; and (6) environmental contamination is induced during chemical processes (Takahashi *et al.,* 1979, *J. Ferment. Tech.* **57**:328-332). Yamada *et al.* (1978, *J. Ferment. Tech.* **56**:484-491) developed a new process for the production of D-amino acids by unsymmetrical transformation of D,L-5'-substituted hydantoin with the D-hydantoinase in microorganisms, followed by treatment with nitrous acid. The products obtained by such chemo-enzymatic process are more economical and convenient. In addition, no measure to ensure optical purity are taken during this process.

Although the chemo-enzymatic process solves the problem in purity in the optical compound, many steps are still required, thereby increasing production costs. In addition, the reaction temperature has to be brought to below 20°C when the step of chemical decarbamoylation is carried out, otherwise D-p-HPG will be further hydrolyzed into 4-hydrobenzaldehyde. For a more convenient production method, many studies have become involved in searching for a microorganism capable of directly hydrolyzing substrates into D-amino acids. Olivieri *et al.* (1979, *Enzyme Microb. Tech*. **1**:201-204) found that the crude extracts of *Agrobacterium radiobater* NRRL B 11291 possess the activity of N-carbamoyl-D-amino acid amidohydrolase, which can further hydrolyze N-carbamoyl-D-amino acid into D-amino acid. Therefore, the D-amino acids can be produced using D-hydantoinase and N-carbamoyl-D-amino acid amidohydrolase in microorganisms (Olivieri *et al.* 1981, *Biotech*. *Bioeng.* **23**:2173-2183). Only one step is required in this method, and the racemizing residues can be completely converted to D-amino acids.

D-hydantoinase (EC 3.5.2.2) is useful in the production of N-carbamoyl-D-amino acids. Currently, microorganisms in which the D-hydantoinase gene has been cloned include *Agrobacterium radiobater*, *Agrobacterium tumefaciens, Bacillus skearothermophilus*, *Bacillus thermoglucosidasius, Pseudomonas putida* DSM 84, etc. The peptide length of D-hydantoinase ranges between 460 and 510 residues.

The two-step enzymatic process is desired for the production of D-amino acids in industries, thereby realizing cost savings for the manufacture. However, the current difficulty is the poor solubility of D,L-*p*-HPH. To overcome this problem, the reaction temperature has to be elevated to 50°C to increase the solubility. In the current cloned D-hydantoinases, however, there is a loss of or decrease in activity at this temperature. Only a few D-hydantoinases cloned from thermophilic *Bacillus* carry sufficient heat-resistance. See, for example, US Patent Nos. 5,523,224, 5,679,571, and 4,912,044. The microorganisms in which the heat-resistant D-hydantoinase has now been cloned include *Bacillus stearothermophilus*, *and Bacillus thermoglucosidasius*. However, some problems in stability and efficiency still exist in these enzymes when they are applied in the industrial process.

Therefore, there is still a need for a thermostable D-hydantoinase, in which the enzyme can maintain activities under high temperature (e.g. 50°C), and perform a preferred catalytic reaction, thereby converting 5'-substituted hydantoin to the desired D-amino acids.

### SUMMARY OF THE INVENTION

The present invention provides a novel D-hydantoinase gene cloned from a thermophilic *Bacillus circulans* and an expression vector to largely express D-hydantoinase in a host. In addition, the present invention discloses the optimal conditions (i.e. divalent ions, pH, temperature) of enzyme activity for conversion of 5'-substituted hydantoin to the corresponding N-carbamoyl-D- and/or -L-α/β-D-amino acids.

It is therefore a primary object of the present invention to provide an isolated nucleic acid molecule. This molecule encodes for a protein consisting of the amino acid sequence set forth in SEQ ID NO:1, wherein the protein has D-hydantoinase activity and retains at least 50% activity after incubation at 50°C for 30 days.

Another object of the present invention is to provide a recombinant vector, which comprises the isolated nucleic acid molecule set forth above and a regulatory sequence.

Still another object of the present invention is to provide a thermostable D-hydantoinase, wherein the amino acid sequence of the D-hydantoinase is set forth in SEQ ID NO:1.

Yet another object of the present invention is to provide a method for preparing a D-amino acid, comprising reacting a 5'-substituted hydantoin with the thermostable D-hydantoinase of the present invention at a pH of 5 to 10 and at a temperature between 30°C and 90°C.

Yet still another object of the present invention is to provide a method for the expression of a thermostable D-hydantoinase in a host cell, comprising (a) introducing the above recombinant vector into the host cell; and (b) culturing the transformed host cell under conditions sufficient to express the thermostable D-hydantoinase. The method can further comprise a step of purifying and recovering the resulting thermostable D-hydantoinase for the large production of thermostable D-hydantoinase of the present invention.

### BRIEF DESCRIPTION OF THE DRAWINGS

The present invention will be more fully understood and further advantages will become apparent when reference is made to the following description of the invention and the accompanying drawings in which:
FIG. 1 is a schematic diagram showing the construct of pSK-A vector;
FIG. 2 is a schematic diagram showing the construct of pSK-B vector;
FIG. 3 is a diagram showing the construct of (A) pQE-*bcidht* vector, and (B) pET-*bcidht* vector;
FIG. 4 is a diagram showing the nucleotide sequence of the thermostable D-hydantoinase with enzyme activity of the present invention (SEQ ID NO:2), and the deduced amino acid sequence thereof (SEQ ID NO:1) ;
FIG. 5 is a diagram showing effects of pH on D-hydantoinase activity of the present invention;
FIG. 6 is a diagram showing the effects of temperature on D-hydantoinase activity in the present invention;
FIG. 7 is a diagram showing the thermostability test of D-hydantoinase on the present invention;
FIG. 8 is a diagram showing effects of metal ion on D-hydantoinase activity of the present invention;
FIG. 9 is a diagram showing the effects of EDTA and 2,6-dipicolinic acid on D-hydantoinase activity in the present invention;
FIG. 10 is a diagram showing the effects of H₂O₂ on D-hydantoinase activity in the present invention;
FIG. 11 is a diagram showing the effects of NaCl on D-hydantoinase activity in the present invention; and
FIG. 12 is a diagram showing the effects of NH₄Cl on D-hydantoinase activity on the present invention.

### DETAILED DESCRIPTION OF THE INVENTION

In accordance with the present invention, there is provided a novel D-hydantoinase gene isolated from a thermophilic *Bacillus circulans,* which the gene encodes for a novel thermostable D-hydantoinase. In one preferred embodiment, the enzyme retains at least 50% D-hydantoinase activity after incubation at 50°C for 30 days. The isolated nucleic acid molecule comprises at least the nucleotide sequence as set forth in SEQ ID NO:2 or the complementary sequence thereof. Those skilled in the art belonging to molecular biology will appreciate that the DNA sequence can be slightly modified using any method known to this art. For example, degenerate codon can be used to replace the corresponding sites without changing the encoded amino acid sequence. Furthermore, additional codons can be added to the 3'- or 5'-end of the DNA sequence or inserted in the DNA sequence, but the activity of the resulting enzyme is only slightly affected, if at all. Genetic engineering methods such as addition, deletion, substitution, etc., are well-known to those skilled in this art and can be seen in Sambrook, et al., Molecular Cloning, A Laboratory Manual, Cold Spring Harbor Laboratory Press, New York, 1989. Those modified sequences are all within the scope of the present invention.

The present invention also provides a recombinant vector comprising the isolated nucleic acid molecule as set forth above and a regulatory sequence. The regulatory sequence used herein can include, for example, plasmid replication origin sequence, selection marker, transcriptional promoter, etc. Suitable promoter sequences for the present invention include, for example, cytomegalovirus (CMV) promoter, simian virus 40 (SV40) early promoter, Rous sarcoma virus (RSV) promoter, etc.

According to the isolated nucleic acid molecule of the present invention, one skilled in this art can express the desired gene product (i.e. D-hydantoinase) in any appropriate manner, with which the D-hydantoinase possesses the amino acid sequence as set forth in SEQ ID NO:1.

The present invention further provides a method for preparing a D-amino acid, comprising reacting a 5'-substituted hydantoin with the thermostable D-hydantoinase of the present invention under suitable conditions, for example, at a pH from 5 to 10 and at a temperature from 30°C to 90°C. The form of thermostable D-hydantoinase used herein can include purified D-hydantoinase, bacteria containing the recombinant vector set forth above or the crude extracts thereof. Suitable substrates (i.e. 5'-substituted hydantoin) include, but are not limited to, hydantoin, dihydrouracil, D,L-*p*-hydroxyphenylhydantoin or D,L-homophenylalanyl-hydantoin. As a result, the conversion of 5'-substituted hydantoin to the corresponding N-carbamoyl-D- and/or -L-α/β-D-amino acids can be achieved using the thermostable D-hydantoinase of the present invention.

The preparation method set forth above further comprises addition of divalent metal ions to enhance the enzyme activity of the present invention. Suitable examples of divalent metal ions include, for example, cobalt (Co²⁺), manganese (Mn²⁺), or nickel (Ni²⁺).

The present invention also provides a method for expression of a thermostable D-hydantoinase in a host cell, comprising introducing the above recombinant vector into the host cell, and then culturing the transformed host cell under conditions sufficient to express the thermostable D-hydantoinase. The method can further comprise a step of purifying and recovering the resulting thermostable D-hydantoinase for the large production of thermostable D-hydantoinase of the present invention.

The host cell used herein is not limited, and can be varied according to the cloned promoter sequence or the industrial application. Such host cells can include prokaryotic cells (e.g. *E*. *coli* or *Bacillus circulans)* or eukaryotic cells (e.g. yeast cell).

The genetic engineering methods used herein, such as DNA modification, cloning and isolation of recombinant vectors, expression and purification of proteins, etc., are well-known to those skilled in this art and can be seen in Ausubel, F.M. *et al.,* Current Protocols in Molecular Biology, New York, 1992; Sambrook *et al*., *supra;* and Davis, L.G., Methods in Molecular Biology, Elsevier, Amsterdam, NL, 1986.

Without intending to limit it in any manner, the present invention will be further illustrated by the following examples.

### EXAMPLE

### Example 1. Cloning of D-hydantoinase gene

### (1) Design of probes for D-hydantoinase gene

The amino acid sequences of the known D-hydantoinases were collected from database, and aligned using GCG program. The DNA sequences with high homology were designed as primers, with the following designated names and sequences:

ADHT-1: 5'-TTCCAGGCGGCATCGACCCGCA-3'

ADHT-2: 5'-GGCGCGGCCGGCCGCTTCACCTTC-3'

ADHT-6: 5'-CCATTCTCCGCATGCACCCATG-3'

Two DNA fragments, 510 bp and 400 bp in length were amplified from chromosomal DNA of *Bacillus circulans by* polymerase chain reaction (PCR). The PCR amplification consisted of 0.075 units *pfu Turbo*™ DNA polymerase (STRTAGENE®, La Jalla, CA) and 1 µM of primers pair of ADH-1 and ADH-2, and another pair of ADH-1 and ADH-6, respectively. PCR was carried out in 4 stages: (1) 94°C for 3 min; (2) 94°C for 1.5 min, 65°C for 2 min, 72°C for 2 min, 25 cycles; (3) 94°C for 1.5 min, 65°C for 1 min, 72°C for 3 min, 1 cycle; and (4) hold at 4°C. The PCR products were sequenced and compared with D-hydantoinases from other organisms in their amino acid sequences. The D-hydantoinase of the present invention showed about 45%-70% identity in amino acid sequence with other D-hydantoinases. The PCR fragment with 510 bp in length was used as a probe for subsequent cloning of *Bacillus circulans* D-hydantoinase.

### (2) Cloning of D-hydantoinase gene

The genomic DNA library from *Bacillus circulans* was hybridized with the ³²P-labeled 510-bp DNA probe. The positive clones were assayed for activities using a selective medium (LB medium containing 1% hydantoin and 0.005% phenol red, pH 7.7). The results show the medium turning from red to bright yellow in color only in the group of pBK-CMV-6 bearing *E*. *coli* XLORL, indicating that this clone possesses the enzyme activity of D-hydantoinase and contains a full-length D-hydantoinase gene which can be expressed in *E. coli.*

### (3) Construction and sequencing of the recombinant vectors pSK-A and pSK-B

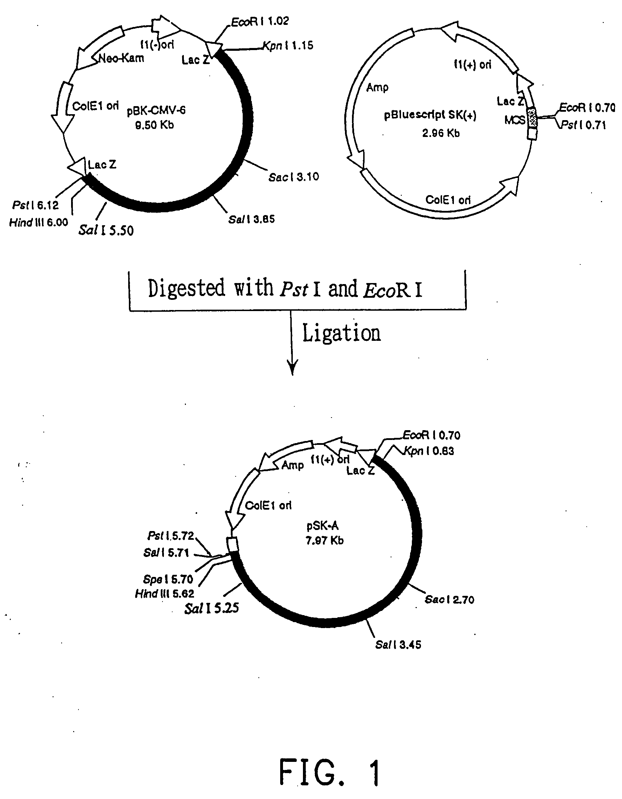

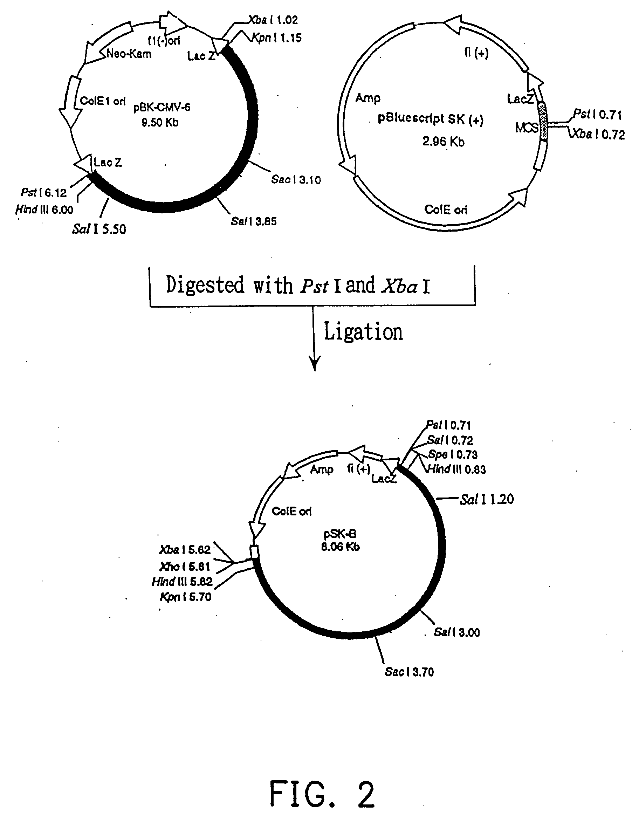

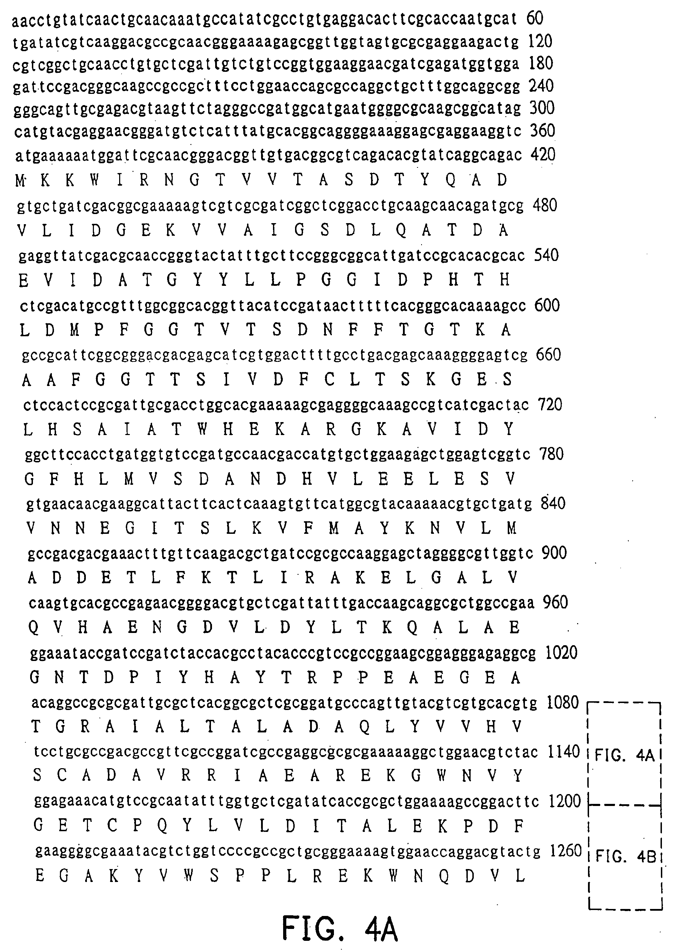

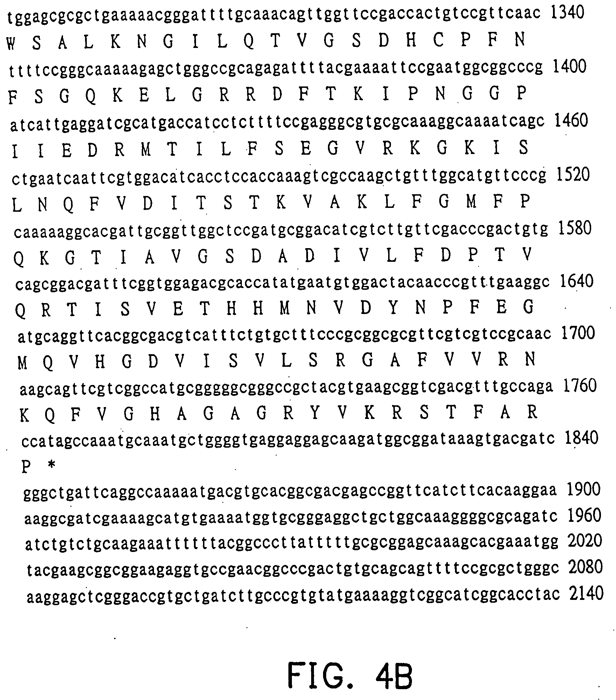

Vector pBK-CMV-6 was digested with the restriction enzyme group of *Eco*RI and *Pst*I, and with another group of *PstI* and *Xba*I. A DNA fragment about 5 kb in length was recovered, and then ligated to pSK(+) vector pre-digested with the same restriction enzymes. The resulting recombinant vectors were designated pSK-A (FIG. 1) and pSK-B (FIG. 2), respectively. Deletion reaction was carried out on both pSK-A and pSK-B vectors using exonuclease III to obtain the deleted fragments with various length. These fragments were ligated, and then transformed into *E. coli* NovaBlue. The transformed clones were selected and sequenced via T7 primer located on the vector. After analysis by DNASTAR software, one full-length D-hydantoinase gene was obtained. This D-hydantoinase gene is 1,386 bp in length, and the 461 amino acids are encoded by the open reading frame (ORF), with a molecular weight of 50,485 Da (Referring to FIG. 4). Other biochemical properties include: G+C content, 57.86%; hydrophobic amino acid content, 37%; polar amino acid content, 24%; iso-electric point (pI) 5.7; and electric charge -10.8 at pH 7.0.

### Example 2. Construction of recombinant vector and expression of D-hydantoinase

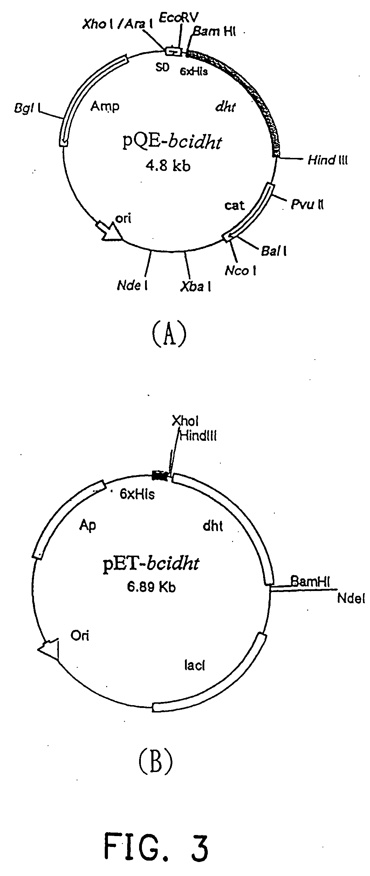

According to the DNA sequence of D-hydantoinase gene, the forward primer was synthesized from the origin of ORF, comprising a *Bam*HI cleavage site. On the other hand, the reverse primer was synthesized from 100 bp downstream of stop codon, comprising a HindIII cleavage site. The D-hydantoinase gene fragments were amplified by PCR, followed by digestion with BamHI and *Hind*III. The treated DNA fragments were ligated into expression vectors pQE-30 and pET-21b, and designated pQE-*bcidht* (FIG. 3(A)) and pET-bcidht (FIG. 3(B)), respectively. The vector pQE-*bcidht* was transformed into *E. coli* NovaBlue to express D-hydantoinase. After culturing the transformants, 1 mM IPTG was added to induce expression when the absorbance of OD₆₀₀ reached 1.0. The induced bacteria were incubated at 28°C for 6 hours to induce the expression of proteins.

### Example 3. Recovery and purification of D-hydantoinase

The culture broth containing the induced bacteria mentioned above was collected. After ultra-sonication, the broth was centrifuged at 13,500 rpm for 20 min. The supernatant was collected and loaded onto the resin containing 6x His tag to adsorb the soluble D-hydantoinase with 6x histidine. The recovered proteins were analyzed by SDS-PAGE, and a protein band at about 51 kDa in size was noted markedly, corresponding to the size of deduced D-hydantoinase.

### Example 4. Ouantification of D-hydantoinase activity

### (1) Reaction pH of D-hydantoinase

The substrate D,L-*p*-HPH was dissolved in various pH buffers at the final concentration of 10 mM. The vector pQE-*bcidht* in *E. coli* NovaBlue, crude extracts thereof or the purified D-hydantoinase of the present invention, were added to each buffer containing substrate, and incubated at 50°C for 10 min. The specific activity of the enzyme was then measured.

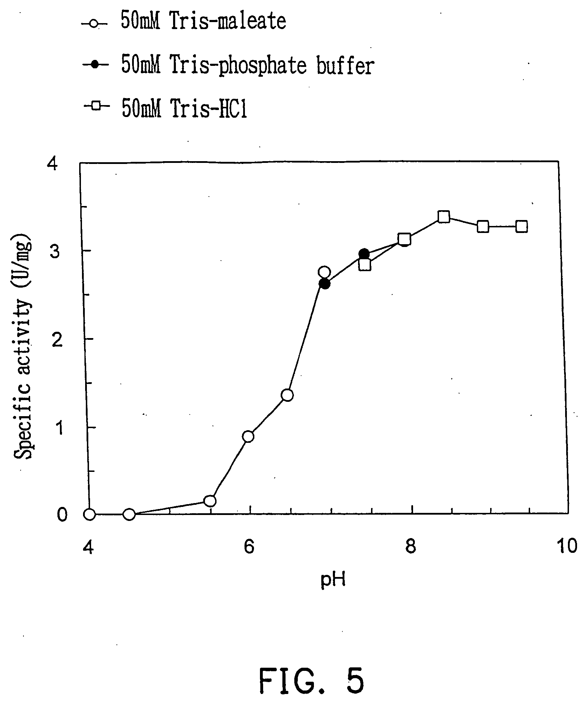

The result is shown in FIG. 5. The lower enzyme activity is found when the solution is acidic. Further, the enzyme loses its entire activity at pH 4.0. By contrast, higher enzyme activity is found when the solution is basic, and the enzyme has the highest specific activity of 3.36 unit/mg at pH 8.5. In addition, referring to FIG. 5, enzyme activity can be found at pH levels ranging from 5 to 10. With the same pH, the specific activity of the enzyme in buffers composed of different components is not, however, identical.

### (2) Reaction temperature of D-hydantoinase

The vector pQE-*bcidht* in *E. coli* NovaBlue, crude extracts thereof or the purified D-hydantoinase of the present invention (containing 500 µM Mn²⁺), was added to 10 mM substrate of D,L-*p*-HPH (dissolved in 50 mM Tris-HCl (pH 8.0)). The mixture was incubated at various temperature (30-90°C) for 10 min. The specific activity of the enzyme was then measured.

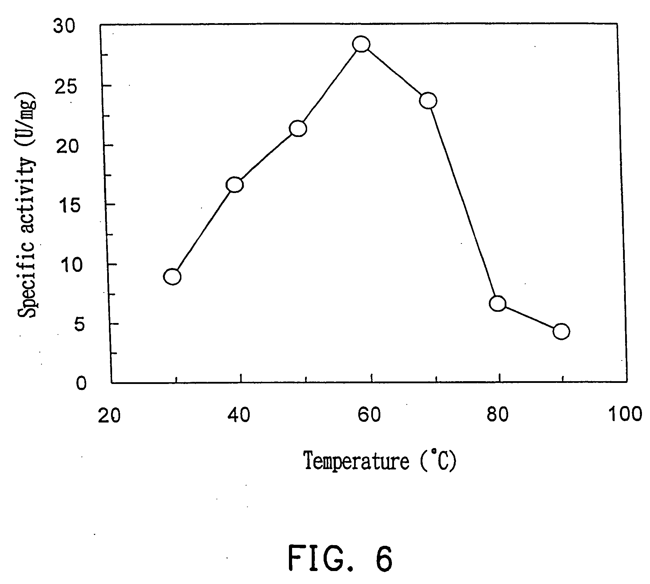

The result is shown in FIG. 6. The specific activity of D-hydantoinase is only 9.0 unit/mg at 30°C. Enzyme activity increases with the elevated temperature, and the enzyme has the highest specific activity of 28.32 unit/mg at 60°C. Above 60°C, enzyme activity suddenly decreases, and with the specific activity of 4.26 unit/mg at 90°C.

### (3) Thermostability of D-hydantoinase

The vector pQE-*bcidht* in *E. coli* NovaBlue, crude extracts thereof or the purified D-hydantoinase of the present invention (47 unit/mg; containing 500 µM Mn²⁺), was incubated at 50°C in a thermostat water bath. The specific activity of the enzyme was measured at various time intervals.

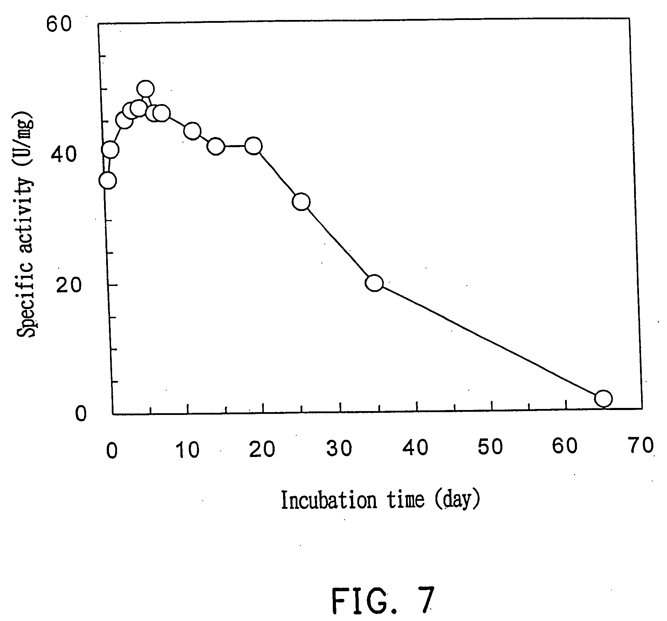

The result is shown in FIG. 7. The half-life of D-hydantoinase is about 35 days. In addition, the enzyme of the present invention retains activity after 60 days incubation at 50°C.

### (4) Relationship between metal ions and enzyme activity

Various divalent metal ions (as set forth in Table 1 below) were added to the vector pQE-*bcidht* in *E*. *coli* NovaBlue, crude extracts thereof or the purified D-hydantoinase of the present invention at 500 µM concentration. After 15 minutes of incubation at room temperature, 10 mM substrate of D,L-*p*-HPH (dissolved in 50 mM Tris-HCl (pH 8.0)) was added. The specific activity of the enzyme was then measured.

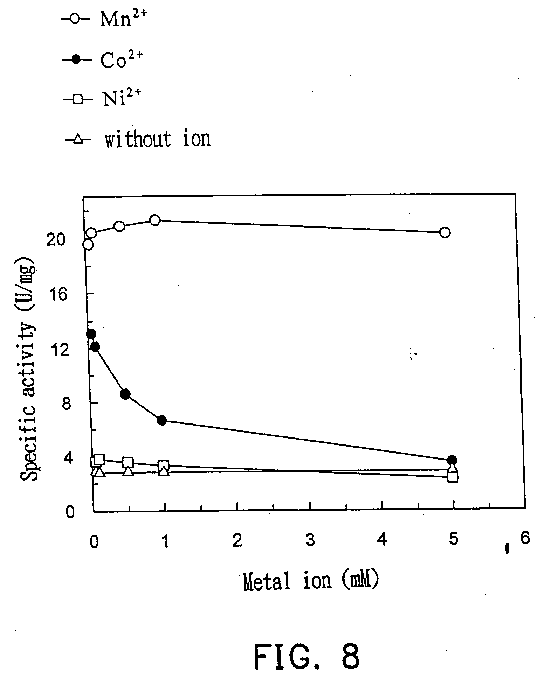

The result is shown in FIG. 8. Cu²⁺ and Zn²⁺ inhibit D-hydantoinase activity at a level of 22% and 50%, respectively; however, Mn²⁺, Co²⁺ and Ni²⁺ enhance the enzyme activity (Table 1). The enzyme activity markedly increases to 7.7 times original activity when the solution contains 500 µM Mn²⁺. In addition, the enzyme activity increases to 4.5 and 1.3 times the original activity in the presence of 50 µM Co²⁺ and 100 µM Ni²⁺, respectively.

**Table 1.**

| Effects of metal ions on D-hydantoinase activity of the present invention | | |
|---|---|---|
| Ion (500 µM) | Specific activity (unit/mg) | Relative activity (%)^{a} |
| No | 2.88 | 100 |
| CoCl₂ | 7.25 | 252 |
| CaCl₂ | 2.53 | 88 |
| CuCl₂ | 2.26 | 78 |
| FeCl₃ | 2.52 | 87.5 |
| FeSO₂ | 2.52 | 87.5 |
| MgSO₄ | 2.69 | 94 |
| MnCl₂ | 22.25 | 773 |
| NiSO₄ | 4.00 | 139 |
| ZnSO₄ | 1.44 | 50 |
| MgCl₂ | 2.62 | 91 |

| | | |
|---|---|---|
| a: Relative activity represents the activity percentage based on that of without adding ions. | | |

### (5) Effects of chelator on D-hydantoinase activity

The vector pQE-*bcidht* in *E*. *coli* NovaBlue, crude extracts thereof or the purified D-hydantoinase of the present invention was mixed with hydrophilic chelator, EDTA, and hydrophobic chelator, 2,6-dipicolinic acid, at 10 µM concentration, respectively. The mixture was incubated for 0-12 hours, and the enzyme activity was measured.

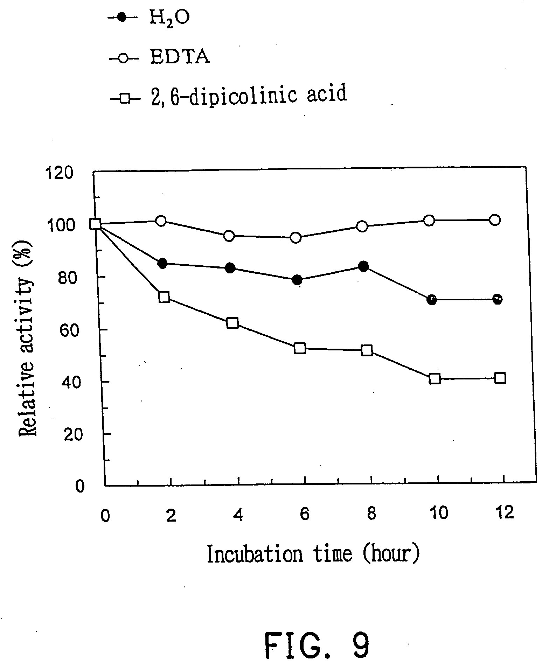

The result is shown in FIG. 9. The enzyme retains 70% activity after incubation with EDTA for 10 hours; whereas it retains only 40% activity after incubation with 2,6-dipicolinic acid for the same period.

### (6) Effects of H₂O₂, NaCl, and NH₄Cl on D-hydantoinase activity

The vector pQE-*bcidht* in *E. coli* NovaBlue, crude extracts thereof or the purified D-hydantoinase of the present invention was incubated at 25°C for 10 min. Afterwards, the catalase was added to the solution to remove residual H₂O₂, and then the enzyme activity was measured. In addition, various concentrations of NaCl and NH₄Cl were added to the solution containing D-hydantoinase. The solution was incubated at room temperature for 10 min, and the enzyme activity was measured.

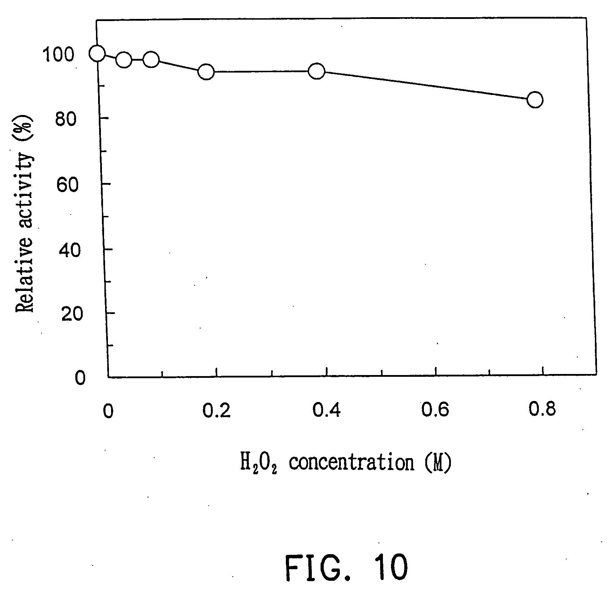

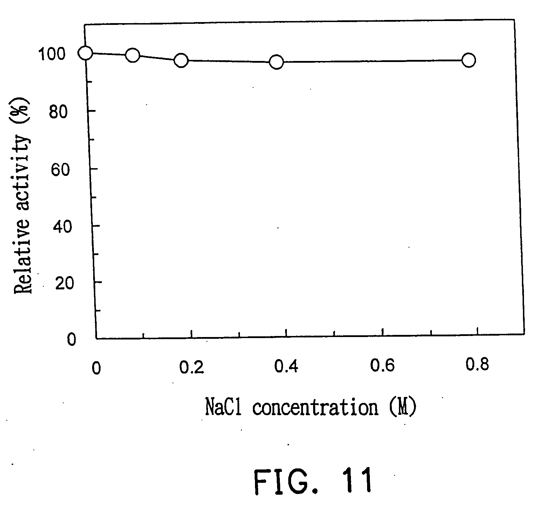

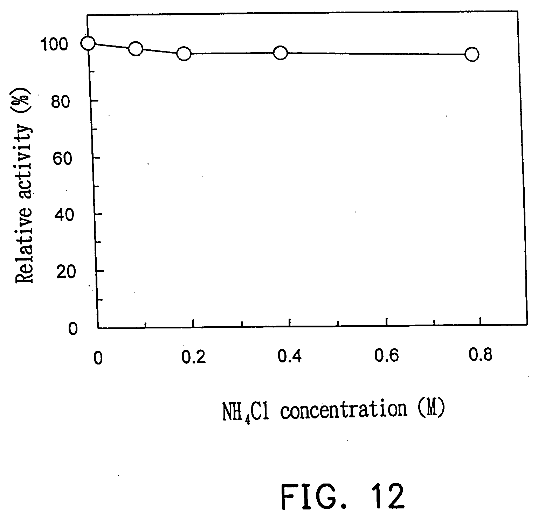

The results are shown in FIGs. 10-12. The enzyme retains 85% activity in the presence of 0.8 M H₂O₂, indicating the high resistance of D-hydantoinase in the present invention to the oxidation of H₂O₂. Moreover, enzyme activity is not affected even when the concentration of NaCl and NH₄Cl is as high as 0.8 M, indicating the D-hydantoinase of the present invention is not inhibited by Na⁺ and NH₄⁺.

### (7) substrate selection of D-hydantoinase

10 mM Dihydrouracil, hydantoin, D,L-*p*-HPH, 5-[2-(methylthio)ethyl]hydantoin, and D,L-homophenylalanyl-hydantoin were used as substrates, respectively. The vector pQE-*bcidht* in *E. coli* NovaBlue, crude extracts thereof or the purified D-hydantoinase of the present invention was reacted with each substrate at 50°C for 10 min. Enzyme activity was then measured.

The result is shown in Table 2. The specific activity of D-hydantoinase for catalysis of dihydrouracil is the highestat 54 unit/mg. Further, the D-hydantoinase of the present invention is also capable of converting hydantoin, D,L-*p*-HPH, 5-[2-(methylthio)ethyl]-hydantoin, D,L-homophenylalanyl-hydantoin, and other 5'-substituted hydantoin to the corresponding N-carbamoyl-D- and/or -L-α/β-D-amino acids thereof.

**Table 2.**

| Substrate selection of D-hydantoinase of the present invention | | |
|---|---|---|
| Substrate (10 mM) | Specific activity (unit/mg) | Relative activity (%)^{a} |
| Dihydrouracil | 54.29 | 100 |
| Hydantoin | 41.43 | 76 |
| D,L-*p*-Hydroxyphenylhydantoin | 47.43 | 87 |
| 5-[2-(Methylthio)ethyl]-hydantoin | 21.48 | 40 |
| D,L-Homophenylalanyl-hydantoin | 45.64 | 84 |

| | | |
|---|---|---|
| a: Relative activity represents the activity percentage based on that of dihydrouracil. | | |

### (8) Conditions of converting D,L-p-HPH by D-hydantoinase

The reaction pH of D-hydantoinase is between 5 and 10. It is also found that the specific activity of the enzyme for catalysis of D,L-*p*-HPH can achieve the maximum level of 47 unit/mg if the enzyme is pre-mixed with Mn²⁺ and incubated in an ice solution for 4 days. The substrate solubility and enzyme activity can be elevated by grinding D,L-*p*-HPH to powder form, dissolving the powdered substrate in 50 mM Tris-HCl (pH 8.0), followed by stirring the solution at room temperature for 2 hours. In addition, a suitable amount of DMSO can also enhance the solubility of substrate D,L-*p*-HPH, and elevate the conversion efficiency from D,L-*p*-HPH to D-*p*-HPH by the D-hydantoinase of the present invention.

While the invention has been shown and described with reference to examples and preferred embodiments, it will be understood by those skilled in the art that modifications can be made without departing from the spirit of the invention.

In summary, the gist of the invention is a nucleic acid encoding a protein having a amino acid sequence as shown in SEQ ID NO:1 and Figures 4A and B, wherein the protein has D-hydantoinase activity and retains at least 50% activity after incubation at 50°C for 30 days. The nucleic acid is preferably selected from a group of molecules consisting of (i) an isolated nucleic acid molecule having a nucleotide sequence as shown in SEQ ID NO:2 or Figures 4A and B; and (ii) an isolated nucleic acid molecule complementary to the aforementioned sequence, that is complementary to SEQ ID NO:2. The nucleic acid is preferably isolated from *Bacillus circulans* and may comprise the usual modifications without departing from the spirit of the invention as long as the protein has D-hydantoinase activity and specifically retains this activity after incubation at 50°C for more than 30 days.

Another aspect of the invention is a recombinant vector comprising a nucleic acid encoding the D-hydantoinase of the invention and a regulatory sequence. The regulatory sequence may preferably comprise an operably linked promoter. In a most preferred embodiment of the second aspect of the invention the recombinant vector is selected from the group consisting of:

SEQUENCE LISTING

<110> National Science Council

<120> THERMOSTABLE D-HYDANTOINASE AND THE APPLICATION
      THEREOF

<130> HL- 1202/UMB

<140> 01110040.1
<141> 2001-04-26

<160> 5

<170> PatentIn Ver. 2.1

<210> 1
<211> 461
<212> PRT
<213> Bacillus circulans

<400> 1
Met Lys Lys Trp Ile Arg Asn Gly Thr Val Val Thr Ala Ser Asp Thr
  1               5                  10                  15

Tyr Gln Ala Asp Val Leu Ile Asp Gly Glu Lys Val Val Ala Ile Gly
             20                  25                  30

Ser Asp Leu Gln Ala Thr Asp Ala Glu Val Ile Asp Ala Thr Gly Tyr
         35                  40                  45

Tyr Leu Leu Pro Gly Gly Ile Asp Pro His Thr His Leu Asp Met Pro
     50                  55                  60

Phe Gly Gly Thr Val Thr Ser Asp Asn Phe Phe Thr Gly Thr Lys Ala
 65                  70                  75                  80

Ala Ala Phe Gly Gly Thr Thr Ser Ile Val Asp Phe Cys Leu Thr Ser
                 85                  90                  95

Lys Gly Glu Ser Leu His Ser Ala Ile Ala Thr Trp His Glu Lys Ala
            100                 105                 110

Arg Gly Lys Ala Val Ile Asp Tyr Gly Phe His Leu Met Val Ser Asp
        115                 120                 125

Ala Asn Asp His Val Leu Glu Glu Leu Glu Ser Val Val Asn Asn Glu
    130                 135                 140

Gly Ile Thr Ser Leu Lys Val Phe Met Ala Tyr Lys Asn Val Leu Met
145                 150                 155                 160

Ala Asp Asp Glu Thr Leu Phe Lys Thr Leu Ile Arg Ala Lys Glu Leu
                165                 170                 175

Gly Ala Leu Val Gln Val His Ala Glu Asn Gly Asp Val Leu Asp Tyr
            180                 185                 190

Leu Thr Lys Gln Ala Leu Ala Glu Gly Asn Thr Asp Pro Ile Tyr His
        195                 200                 205

Ala Tyr Thr Arg Pro Pro Glu Ala Glu Gly Glu Ala Thr Gly Arg Ala
    210                 215                 220

Ile Ala Leu Thr Ala Leu Ala Asp Ala Gln Leu Tyr Val Val His Val
225                 230                 235                 240

Ser Cys Ala Asp Ala Val Arg Arg Ile Ala Glu Ala Arg Glu Lys Gly
                245                 250                 255

Trp Asn Val Tyr Gly Glu Thr Cys Pro Gln Tyr Leu Val Leu Asp Ile
            260                 265                 270

Thr Ala Leu Glu Lys Pro Asp Phe Glu Gly Ala Lys Tyr Val Trp Ser
        275                 280                 285

Pro Pro Leu Arg Glu Lys Trp Asn Gln Asp Val Leu Trp Ser Ala Leu
    290                 295                 300

Lys Asn Gly Ile Leu Gln Thr Val Gly Ser Asp His Cys Pro Phe Asn
305                 310                 315                 320

Phe Ser Gly Gln Lys Glu Leu Gly Arg Arg Asp Phe Thr Lys Ile Pro
                325                 330                 335

Asn Gly Gly Pro Ile Ile Glu Asp Arg Met Thr Ile Leu Phe Ser Glu
            340                 345                 350

Gly Val Arg Lys Gly Lys Ile Ser Leu Asn Gln Phe Val Asp Ile Thr
        355                 360                 365

Ser Thr Lys Val Ala Lys Leu Phe Gly Met Phe Pro Gln Lys Gly Thr
    370                 375                 380

Ile Ala Val Gly Ser Asp Ala Asp Ile Val Leu Phe Asp Pro Thr Val
385                 390                 395                 400

Gln Arg Thr Ile Ser Val Glu Thr His His Met Asn Val Asp Tyr Asn
                405                 410                 415

Pro Phe Glu Gly Met Gln Val His Gly Asp Val Ile Ser Val Leu Ser
            420                 425                 430

Arg Gly Ala Phe Val Val Arg Asn Lys Gln Phe Val Gly His Ala Gly
        435                 440                 445

Ala Gly Arg Tyr Val Lys Arg Ser Thr Phe Ala Arg Pro
    450                 455                 460

<210> 2
<211> 2100
<212> DNA
<213> Bacillus circulans

<400> 2
aacctgtatc aactgcaaca aatgccatat cgcctgtgag gacacttcgc accaatgcat 60
tgatatcgtc aaggacgccg caacgggaaa agagcggttg gtagtgcgcg aggaagactg 120
cgtcggctgc aacctgtgct cgattgtctg tccggtggaa ggaacgatcg agatggtgga 180
gattccgacg ggcaagccgc cgctttcctg gaaccagcgc caggctgctt tggcaggcgg 240
gggcagttgc gagacgtaag ttctagggcc gatggcatga atggggcgca agcggcatag 300
catgtacgag gaacgggatg tctcatttat gcacggcagg ggaaaggagc gaggaaggtc 360
atgaaaaaat ggattcgcaa cgggacggtt gtgacggcgt cagacacgta tcaggcagac 420
gtgctgatcg acggcgaaaa agtcgtcgcg atcggctcgg acctgcaagc aacagatgcg 480
gaggttatcg acgcaaccgg gtactatttg cttccgggcg gcattgatcc gcacacgcac 540
ctcgacatgc cgtttggcgg cacggttaca tccgataact tttcacgggg cacaaaagcc 600
gccgcattcg gcgggacgac gagcatcgtg gacttttgcc tgacgagcaa aggggagtcg 660
ctccactccg cgattgcgac ctggcacgaa aaagcgaggg gcaaagccgt catcgactac 720
ggcttccacc tgatggtgtc cgatgccaac gaccatgtgc tggaagagct ggagtcggtc 780
gtgaacaacg aaggcattac ttcactcaaa gtgttcatgg cgtacaaaaa cgtgctgatg 840
gccgacgacg aaactttgtt caagacgctg atccgcgcca aggagctagg ggcgttggtc 900
caagtgcacg ccgagaacgg ggacgtgctc gattatttga ccaagcaggc gctggccgaa 960
ggaaataccg atccgatcta ccacgcctac acccgtccgc cggaagcgga gggagaggcg 1020
acaggccgcg cgattgcgct cacggcgctc gcggatgccc agttgtacgt cgtgcacgtg 1080
tcctgcgccg acgccgttcg ccggatcgcc gaggcgcgcg aaaaaggctg gaacgtctac 1140
ggagaaacat gtccgcaata tttggtgctc gatatcaccg cgctggaaaa gccggacttc 1200
gaaggggcga aatacgtctg gtccccgccg ctgcgggaaa agtggaacca ggacgtactg 1260
tggagcgcgc tgaaaaacgg gattttgcaa acagttggtt ccgaccactg tccgttcaac 1320
ttttccggcc aaaaagagct gggccgcaga gattttacga aaattccgaa tggcggcccg 1380
atcattgagg atcgcatgac catcctcttt tccgagggcg tgcgcaaagg caaaatcagc 1440
ctgaatcaat tcgtggacat cacctccacc aaagtcgcca agctgtttgg catgttcccg 1500
caaaaaggca cgattgcggt tggctccgat gcggacatcg tcttgttcga cccgactgtg 1560
cagcggacga tttcggtgga gacgcaccat atgaatgtgg actacaaccc gtttgaaggc 1620
atgcaggttc acggcgacgt catttctgtg ctttcccgcg gcgcgttcgt cgtccgcaac 1680

aagcagttcg tcggccatgc gggggcgggc cgctacgtga agcggtcgac gtttgccaga 1740
ccatagccaa atgcaaatgc tggggtgagg aggagcaaga tggcggataa agtgacgatc 1800
gggctgattc aggccaaaaa tgacgtgcac ggcgacgagc cggttcatct tcacaaggaa 1860
aaggcgatcg aaaagcatgt gaaaatggtg cgggaggctg ctggcaaagg ggcgcagatc 1920
atctgtctgc aagaaatttt ttacggccct tatttttgcg cggagcaaag cacgaaatgg 1980
tacgaagcgg cggaagaggt gccgaacggc ccgactgtgc agcagttttc cgcgctgggc 2040
aaggagctcg ggaccgtgct gatcttgccc gtgtatgaaa aggtcggcat cggcacctac 2100

<210> 3
<211> 22
<212> DNA
<213> Artificial Sequence

<220>
<223> Description of Artificial Sequence:Primer for PCR

<400> 3
ttccaggcgg catcgacccg ca 22

<210> 4
<211> 24
<212> DNA
<213> Artificial Sequence

<220>
<223> Description of Artificial Sequence:Primer for PCR

<400> 4
ggcgcggccg gccgcttcac cttc 24

<210> 5
<211> 22
<212> DNA
<213> Artificial Sequence

<220>
<223> Description of Artificial Sequence:Primer for PCR

<400> 5
ccattctccg catgcaccca tg 22

## Claims

1. An isolated nucleic acid molecule which encodes for a protein having the amino acid sequence as shown in SEQ ID NO:1 and Figures 4A and B, wherein the protein has D-hydantoinase activity and retains at least 50% activity after incubation at 50°C for 30 days.

2. The isolated nucleic acid molecule as claimed in claim 1, wherein the nucleic acid is selected from the group consisting of:
(i) an isolated nucleic acid molecule having the nucleotide sequence as shown in SEQ ID NO:2 and Figures 4A and B; and
(ii) an isolated nucleic acid molecule complementary thereto.

3. The isolated nucleic acid molecule as claimed in claim 2, wherein the nucleic acid is isolated from *Bacillus circulans.*

4. A recombinant vector comprising the isolated nucleic acid molecule as claimed in claim 1 and a regulatory sequence.

5. The recombinant vector as claimed in claim 4, wherein the regulatory sequence comprises an operably linked promoter.

6. The recombinant vector as claimed in claim 5, wherein the recombinant vector is selected from the group consisting of:
(i) pBK-CMV-6 having the construct as shown in figure 1, Deposit Number: PTA-2553; and
(ii) pQE-bcidht which has the construct as shown in figure 3(A), Deposit Number: PTA-2554.

7. A thermostable D-hydantoinase, wherein the amino acid sequence of the D-hydantoinase is set forth in SEQ ID NO:1.

8. The thermostable D-hydantoinase as claimed in claim 7, wherein the D-hydantoinase is expressed by the recombinant vector as claimed in claim 6.

9. A method for preparing a D-amino acid, comprising reacting a 5'-substituted hydantoin with the thermostable D-hydantoinase as claimed in claim 7 at a pH from 5 to 10 and at a temperature from 30°C to 90°C.

10. The method as claimed in claim 9, wherein the form of the thermostable D-hydantoinase comprises a purified D-hydantoinase, a bacterium containing the recombinant vector of claim 6 or the crude extracts thereof.

11. The method as claimed in claim 9, wherein the 5' -substituted hydantoin comprises hydantoin, dihydrouracil, D,L-*p*-hydroxyphenylhydantoinorD,L-homophenylalanylhydantoin.

12. The method as claimed in claim 9, further comprising adding a divalent metal ion to enhance the activity of the D-hydantoinase.

13. The method as claimed in claim 12, wherein the divalent metal ion is selected from the group consisting of cobalt, manganese, and nickel.

14. A method for expression of a thermostable D-hydantoinase in a host cell, comprising the steps of:
(a) introducing the recombinant vector of claim 6 into said host cell; and
(b) culturing said transformed host cell under conditions sufficient to express the thermostable D-hydantoinase.

15. A method for producing a thermostable D-hydantoinase, comprising the steps of:
(a) introducing the recombinant vector of claim 6 into a host cell;
(b) culturing said transformed host cell under conditions sufficient to produce the thermostable D-hydantoinase; and
(c) purifying and recovering the thermostable D-hydantoinase.

16. The method as claimed in claim 15, wherein the step of purifying and recovering is performed by column chromatography.

17. The method as claimed in claim 14 or claim 15, wherein said host cell comprises a prokaryotic cell or an eukaryotic cell.

18. The method as claimed in claim 17, wherein the prokaryotic cell is from *E.coli* or *Bacillus circulans.*

19. The method as claimed in claim 17, wherein the eukaryotic cell comprises yeast cell.
